# EUROPEAN PATENT APPLICATION

(11) **EP 0 647 427 A1**
(43) Date of publication of application: **12.04.1995**
(21) Application number: 94107857.8
(22) Date of filing: 20.05.1994
(51) Int. Cl.: A61B 5/08, A61B 5/087

(54) **Breath flowmeter apparatus and health maintenance system**

(30) Priority: 08.10.1993 JP 252701/93
(71) Applicant: CHEST M.I. INC, Tokyo (JP)
(72) Inventor: Takishima, Tamotsu, Tokyo (JP); Komoda, Masaji, Tokyo (JP)
(74) Representative: Münich, Wilhelm, Dr.

(57) **Abstract**

This application discloses a portable type breath flowmeter apparatus and an asthma management system. The apparatus comprises a memory for storing a measured expiration data together with date and time period. Further, the system comprises a breath flowmeter apparatus 1 having a memory 23 and a transmitting interface (transmitting means) 21, and an asthma data management apparatus 30 having a host computer (analysis means) 31 and a printer (output means) 32. Where the patient measures an expiration the breath flow data are kept stored together with the measuring time information. The data is sent via the transmitting interface 21 to the host computer and the analysis graph is outputted from the printer 32.

## Description

### BACKGROUND OF THE INVENTION

### FIELD OF THE INVENTION

The present invention relates to breath flowmeter apparatuses and asthma management system suitable for diagnosis and management of asthma.

### DESCRIPTION OF THE PRIOR ART

The peak exipratory flow rate (PEFR) analyzed from forced expiratory maneuver tends to gradually decrease before arising of an asthma attack. Therefore, if such tendency of decreasing is grasped by measuring PEFR everyday, a high dose of a predetermined medicine such as bronchodilator is given to the patient before the asthma attack to prevent an attack of the patient.

In such circumstances, recently, a portable and mechanical type breath flowmeter apparatus for measuring the PEFR at patient's home has been developed.

The asthma patient can measure, by using the breath flowmeter apparatus, PEFR everyday, for example, in the morning, the daytime and the evening, record the measured values or a trend graph thereof into a asthmatic diary and grasp the condition of the disease objectively. The patient takes the medicine in accordance with the medical doctor's instruction based on decision of the zone determination such as green, yellow, red and the like, that is, a lung function assessment information showing an extent of PEFR relative to the reference value of the patient previously set by the medical doctor, thereby the patient prevents occurring of a asthma attack and controls his disease condition. The medical doctor investigates the patient's condition and the asthmatic diary carried with the patient at every several week basis so as to perform an adequate diagnosis and treatment. The medicine for preventing the asthma attack, for example, bronchodilator may preferably be dosed to the patent at minimum but appropriate quantity depending on the disease condition because such medicine gives an adverse reaction to the patient.

On the other hand, keeping the asthmatic diary is cumbersome and time-consuming, and the youngster and eldery patients sometimes mis-records or records forged values without measurement. In such case, if the medical doctor diagnoses the patients on the basis of the diaries, danger of an erroneous diagnosis may arise, this tends to cause death of asthma attack or the adverse reaction by the excess dosage of the medicine.

For this reason, for the asthma management of the patients, it is extremely important to record PEFR exactly and to diagnose correctly based on the recorded data.

The measurement of PEFR is an inspection depending on effort of the patient in terms of the expiratory physiology, thus, dispersion tends to arise in the measured data. Thus, if measuring the volume of air expired in one second from maximum inspection (FEV1), which is hardly affected by an extent of effort of the patient, is performed together with the measurements depending on the patient's effort, more correct assessment of the disease condition is achieved.

However, in the conventional breath flowmeter apparatus field, there has not been found the apparatus of a portable type capable of measuring both of PEFR and FEV1 as measured data of a forced expiratory maneuver. Recording the daily measured values into the asthmatic diary is a burden for the patient, and this tends to cause incorrect recordation of the measured values. It is cumbersome also for the medical doctor to properly manage those measured data and utilize the managed data in the diagnosis.

A first object of the invention is to provide a breath flowmeter apparatus of a portable type capable of precisely measuring and recording at least PEFR. A second object of the invention is to provide an asthma management system which securely performs the asthma management of the patient with reduction of burdens of the patient and the medical doctor.

### SUMMARY OF THE INVENTION

To achieve the first object of the present invention, the breath flowmeter apparatus of the present invention comprising: measuring means for measuring expiration data on the basis of a breath blown into a breath breath cylinder at each measuring operation; display means for displaying the expiration data; storage means for storing the expiration data together with measuring time information containing a date and a time period when the measurement is performed; and a battery for supplying a power source to each part of the apparatus. The apparatus may be configurated such that, where the measurements by the measuring means are performed a plurality of times during a same time period on a same date, the expiration datum indicative of the maximum value is selected out of the thus obtained plurality of expiration data as an expiration datum of the time period so as to be stored in the storage means. The measured data may include at least PEFR. Further, the breath cylinder may be configurated such that the breath cylinder is detachable to the body of the apparatus.

To achieve the second object of the present invention, an asthma management system of the present invention has a breath flowmeter apparatus and an asthma data management apparatus, and is characterized in that the flowmeter apparatus comprises a memory for storing an expiration datum measured at every measuring operation together with measuring time information containing a date and a time period when the measurement is performed, and transmission means for transmitting together with the measuring time information the expiration data stored in the memory to the asthma data management apparatus, and that the asthma data management apparatus includes analysis means for analyzing expiration flow data transmitted from the transmission means, and output means for outputting the analysis result by the analysis means.

According to thus constructed breath flowmeter apparatus, since expiration data can be read out of the storage means on a date and a time period basis, a suitable control for the asthmatic patient is obtained. Since the breath flow data ranging several weeks can be stored together with measuring time information, a correspondence between the measuring time and the measured data is clarified, and the mis-recording or forging data by the patient is prevented, and therefore, correct data can be obtained. Since the measured data is not required to be recoreded into a diary etc., a burden on the patient can be reduced. Since the measured data stored in the storage means can be read into a host computer provided in the hospital and be analyzed, a burden on the medical doctor is also reduced. With the battery-driving used, a portable type is realized to facilitate the measurement at home. The stable measurement can be obtained because the maximum value is emplyed among the operational results of a plurality of times' breaths as a breath flow amount. This also ensures that the patient himself grasps the disease condition objectively to respond appropriately to the instruction of the medical doctor. With the structure in which the breath cylinder can be releasably mounted to the apparatus main body, the breath cylinder can be released from the apparatus main body when washing it, thus facilitating the washing of the breath cylinder.

According to the asthma management system configurated as above, when the patient measures the breath flow amount by using the breath flowmeter apparatus, the memory stores the measured expiration data together with the measuring time information. When the measured expiration data stored by a transmission means together with the measuring time information are transferred to the asthma data management apparatus, the analysis means analyzes the expiraiton data transferred by the transmission means, and then the output means outputs the analyzed result by the analysis means.

With this configuration, since recording the measured expiration data into the diary is not required, a burden on the patient can be reduced. Since the measured data can be input directly into the host computer by the asthma data management apparatus and analyzed therein, the burden on the medical doctor is reduced. Since the data management ranging several years can be realized by the host computer, a secure asthma data management for the asthmatic patient is realized.

### BRIEF DESCRIPTION OF THE DRAWINGS

Fig. 1 is a front-side perspective view showing one embodiment of a breath flowmeter apparatus of the present invention;
Fig. 2 is a backside exploded view of the breath flowmeter apparatus in Fig. 1;
Fig. 3 is a backside exploded perspective view of the breath flowmeter apparatus in Fig. 1;
Fig. 4 is a sectional view of a nozzle-releasable button;
Fig. 5 is a vertical sectional view thereof;
Fig. 6 is a block diagram showing a control system of the apparatus;
Figs. 7 to 15 are views showing display examples of the apparatus;
Fig. 16 is a block diagram showing one embodiment of an asthma management system of the present invention; and
Fig. 17 is a graph showing an output example by a printer of an asthma data management apparatus of the system.

### DESCRIPTION OF THE PREFERRED EMBODIMENTS

Embodiments of the invention are described in detail referring to the drawings.

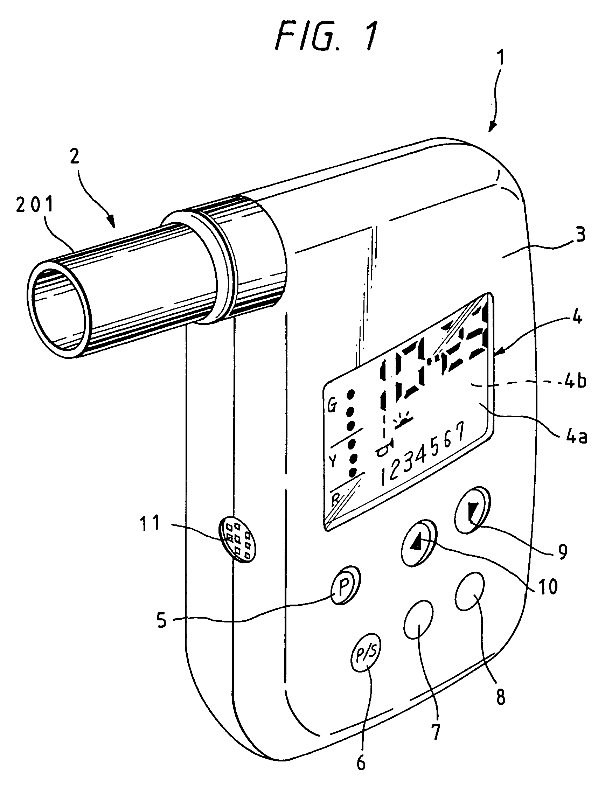

Fig. 1 is a front-side perspective view showing one embodiment of a breath flowmeter apparatus of the present invention.

An apparatus 1 comprises a breath cylinder 2 which is releasably formed in a cylindrical shape releasably configurated and used for introducing breath of the patient, and a main body 3 which is in box shape and integraly incorporated on a lower portion thereof. The main body 3 is provided on its front-side center with a display 4, on its front-side lower portion with a power switch 5, a select switch 6, a register switch 7, a display switch 8, a first shift switch 9 and second shift switch 10 respectively. The main body 3 is further provided on its side-surface with an output terminal 11 used for a communication interface 21 as a transmission means which is described later.

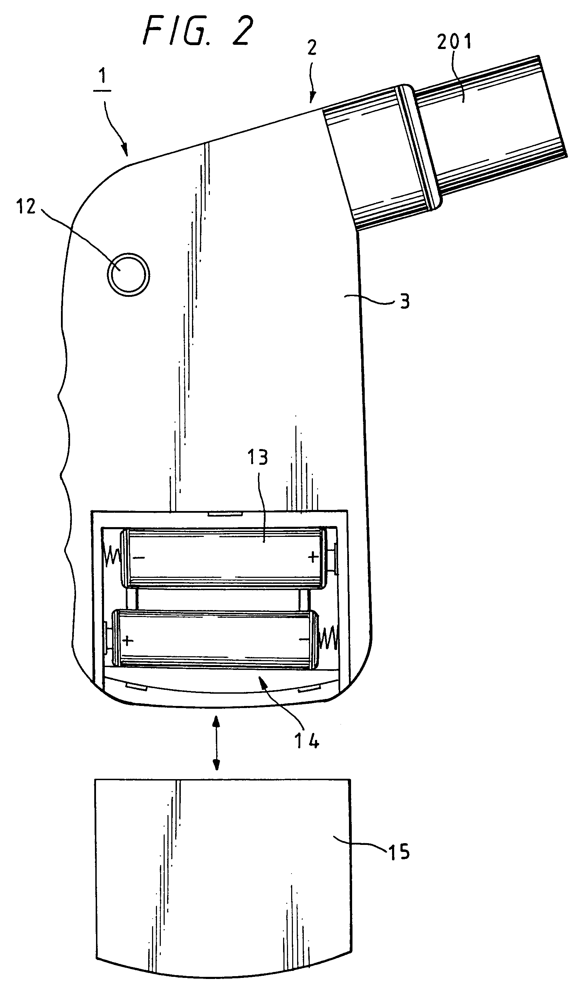

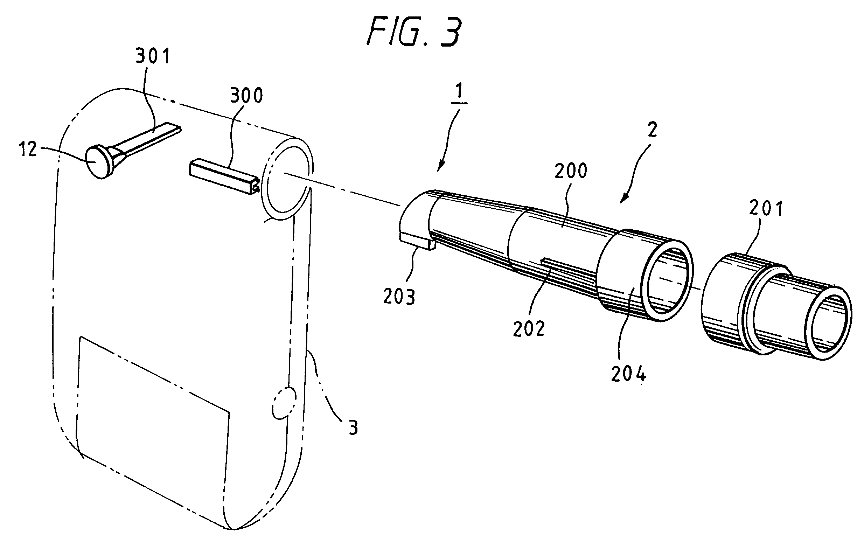

Fig. 2 is a backside exploded view of the apparatus 1, and Fig. 3 is a backside exploded perspective view of the apparatus 1.

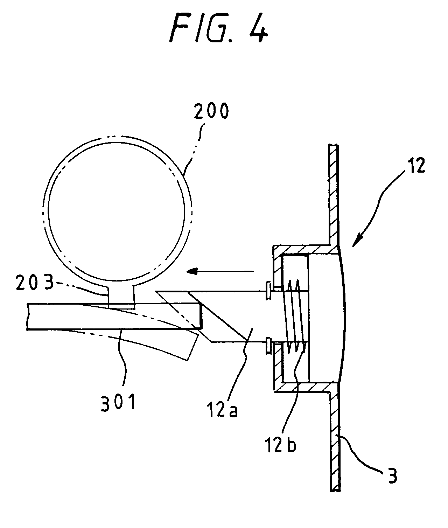

In Fig. 2, the apparatus 1 comprises a nozzle-releasable button 12 for releasing lock of the breath cylinder 2, a battery container 14 for containing batteries 13, and a battery cover 15 for covering the battery container 14. In Fig. 3, the breath cylinder 2 includes a sensor nozzle 200 in a cylindrical shape releasable to the main body 3, and a mouthpiece 201 in a cylindrical shape releasable to the sensor nozzle 200. The sensor nozzle 200 is provided on its side-surface with a guide rail 202, on its lower surface with a stopper 203 each in a protruded manner, and provided with a brim 204 on mouthpiece 201 side. In Fig. 3, inside the main body 3 there are provided a guide groove 300 for guiding the guide rail 202, and a lever 301 which is locked to prevent the stopper 203 from slipping out. In Fig. 4, the nozzle-releasable button 12 includes a releasing shaft 12a whose tip end is formed in taper, and a spring 12b forcing the nozzle-releasable button 12 to the outside.

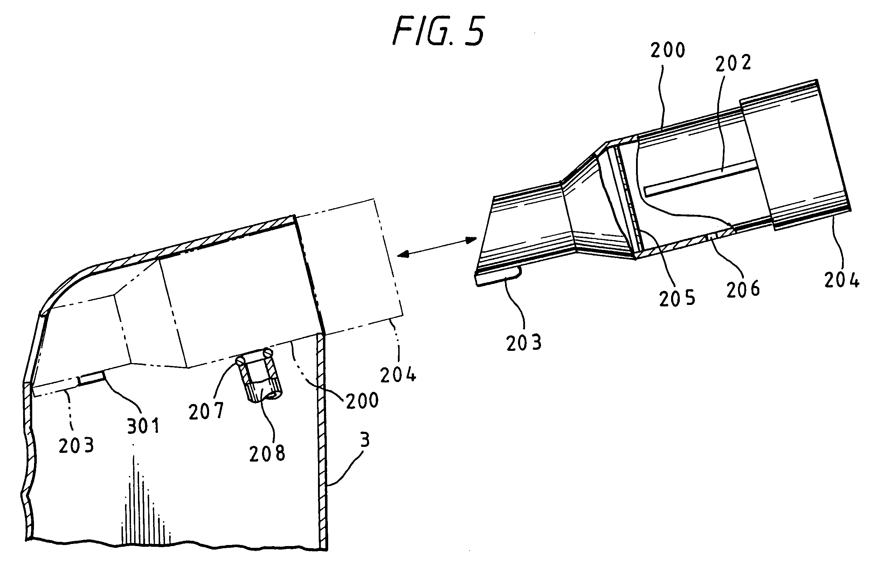

Next, attaching and detaching the breath cylinder 2 will further be described referring to Figs. 4 and 5. Fig. 4 is a partial sectional view of the nozzle-releasable button 12 and Fig. 5 is a vertical sectional view thereof.

In state where the breath cylinder 2 is kept separated from the main body 3, the lever 301 is in horizontal state without bent as shown by solid lines in Fig. 4, and the nozzle-releasable button 12 is kept forced to front surface-side of the main body 3 by the lever 301. When loading the breath cylinder 2 on the main body 3 , the breath cylinder 2 is inserted until the brim 204 of the sensor nozzle 200 contacts on the main body 3 so that the guide rail 202 of the sensor nozzle 200 can enter the guide groove 300 of the main body 3. At this time, the stopper 203 is inserted with the lever 301 temporarily bent. After completely inserting the breath cylinder 2, as shown by solid lines in Fig. 4, the lever 301 is returned to a horizontal attitude without bent, and as shown by phantom lines in Fig. 5 the stopper 203 is locked by the lever 301 for preventing slipping-out. When releasing the breath cylinder 2 from the main body 3, the nozzle-releasable button 12 is depressed, as shown phantom lines in Fig. 4. The lever 301 is bent in a lower direction because of the taper shape of tip end of the releasable shaft 12a to release lock of the breath cylinder 2, which therefore is released from the main body 3 by holding the breath cylinder 2 in hand. Thus, the breath cylinder 2 can be released from the main body 3 and washed.

As shown in Fig. 5, the sensor nozzle 200 is provided with a flexible valve 205 and a vent hole 206. The flexible valve 205 is made of, for example, several polyester thin plates with one end of each of the thin plates being fixed on an inside surface of the sensor nozzle 200, whereby the thin plates are bent in accordance with expiratory flow so as to open or close the flow passage. The vent hole 206 is, when the sensor nozzle 200 is mounted on the main body 3, connected to a trasnducer (not shown) via and O-ring 207 and a vent pipe 208 both provided in the main body 3, whereby a pressure within the sensor nozzle 200 varied with the expiratory flow of the patient is detected by a pressure transducer.

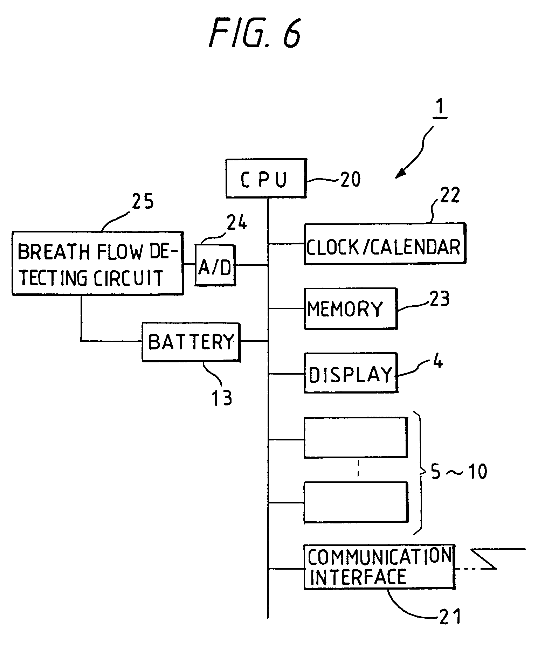

Fig. 6 is a block diagram showing a control system of the apparatus 1.

The apparatus 1 includes a CPU 20 for controlling each part of the apparatus 1. The CPU 20 is connected with the display 4, the various switches 5 to 10, the communication interface 21, and further connected with a expiratory flow detecting circuit 25 through a clock / calendar 22, a memory 23 and an A/D converter 24. The batteries 13 are connected to the expiratory flow detecting circuit 25 and each part of the apparatus 1.

The expiratory flow detecting circuit 25, includes a expiratory flow converting means having a pressure transducer (not shown) for detecting internal pressure of a sensor nozzle 200, wherein the converting means detects, a current corresponding to the patient's expiratory flow rate on the breath cylinder 2 , and outputs thus detected value. The converting means may be configurated in another way as long as the expiratory flow rate into electric signals.

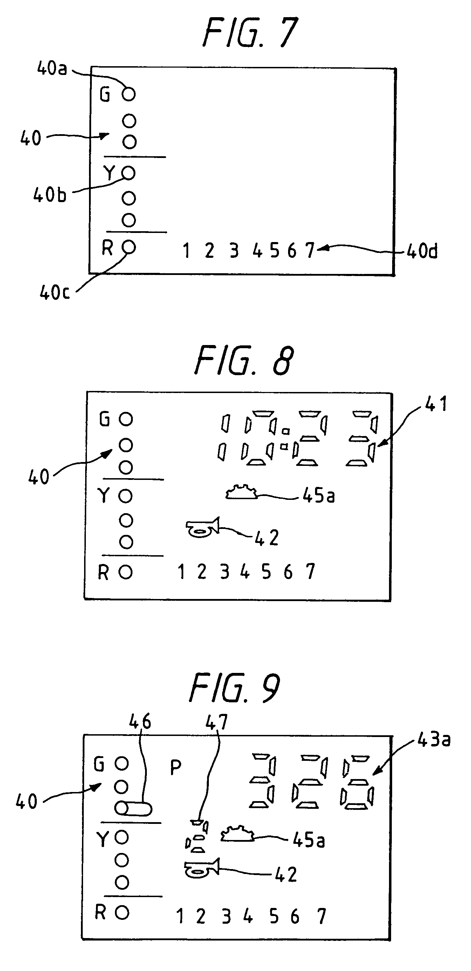

The CPU 20 performs a predetermined operation and the display-control as shown in display in Figs. 7 to 15, where the control proceeds for each part of the apparatus 1 to realize functions such as "auto-power-off function", "toggle operation function", and "battery alarm display function" which are described later.

The CPU 20 takes in detected value from the expiratory flow detecting circuit 25 through the A/C converter 24 to obtain PEFR and FEV1, and stores its computed result together with the measuring time information (date and time when measurement is performed ) sent from the clock / calendar 22 into the memory 23. The measurements are performed several times every day, and the operational result of each measurement is sequentially stored in the memory 23. The CPU 20 scroll-displays together with the measuring time information the measured data stored in the memory 23 on a liquid crystal panel 4b of the display 4 in accordance with depression of the first and second shift switches 9 and 10 after depressing the display switch 8. Moreover, the CPU 20 is capable of transmitting measured data sequentially stored in the memory 23 to a host computer provided in the hospital, for example, through a RS-232C type communication interface 21, such host computer being described later. With this arrangement, a burden to the patient is lowered since the patient is not required to record the measured data into the diary and the like and to submit it to the hospital. The burden imposed on the medical doctor is also lightened since the doctor can read the corresponding measured data from the host computer and immediately perform the analysis thereof.

The display 4 is arranged such that a transparent plate 4a and a liquid crystal panel 4b overlap each other, the transparent plate 4a forming a front-side and the liquid crystal panel 4b forming a and rear-side respectively. In Fig. 7, the transparent plate 4a on front-side is printed thereon with a plurality of (for example, seven) color marks 40 (40a to 40c) along upper and lower directions on left-side, and numerals 40d of 1 to 7 on lower-side. The color marks 40a to 40c are provided for indicating zones to which the measured data belong so as to facilitate the management according to the asthma condition. The zones are green zone, yellow zone, and red zone, and in the drawing, a zone shown by G is provided with three color marks 40a, a zone shown by Y with three color marks 40b, a zone shown by R with one color mark 40c.

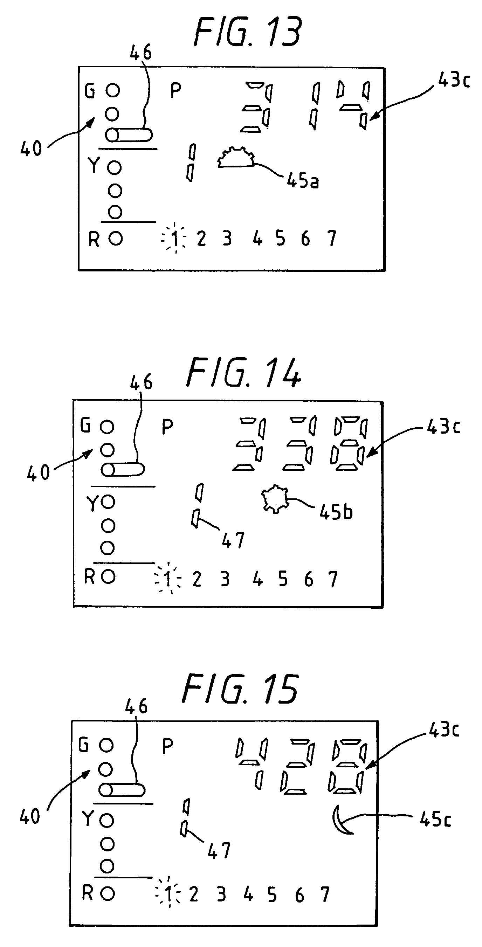

When the power switch 5 is depressed, as shown in Fig. 8, the present time 41 is displayed on upper right of the liquid crystal panel 4b, and simultaneously a trumpet mark 42 showing availability of measurement and a measuring time period 45 (45a) are displayed on center thereof. The measuring time period 45 (45a) represents morning when a mark indicative of the sun whose upper part appears above the horizontal line is displayed as shown in Fig. 8, daytime when a mark 45b showing the entire sun is displayed as in Fig. 14, and evening when a mark 45c indicative of the moon is displayed on right-side of the display as shown in Fig. 15. When the power switch 5 is depressed to turn ON the power supply, thereafter if any of switches 5 to 10 is not depressed for two minutes, then the power supply is automatically turned OFF by the CPU 20 control, which is called as an "auto-power-off function" described later. After once depressing the power switch 5 to turn ON the power supply, then with the power switch 5 again depressed, the power supply is turned OFF by control of the CPU 20, which is called as "a toggle operation function". When a voltage of the battery 13 is lowered equal to or less than a predetermined voltage (for example, 2.2 V), the display dot of the liquid crystal panel 4b flickers by CPU 20 control, which is called as "a battery alarm display function". In the event of extracting the batteries 13 from the battery container 14 arranged on the backside surface of main body 3, stored contents in the memory 23 is saved during more than the specified time period (for example, one hour).

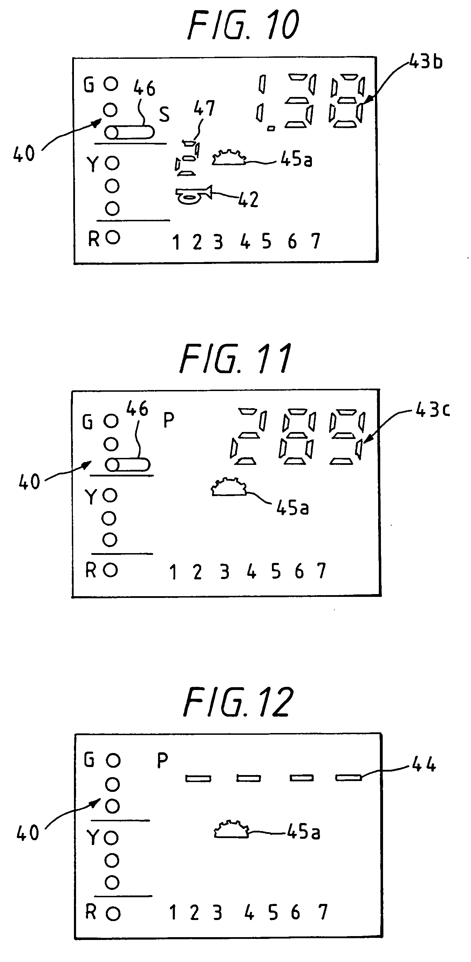

The select switch 6 is provided for selecting measuring items of PEFR and FEV1, where "PEFR" represents the maximum flow rate (L/min) of a forced expiratory manuever, and in this embodiment the measurement can be performed in a range of 50 to 850 (L/min), and "FEV1" represents a volume (L) of air expired in one second from maximum inspiration, and in this embodiment the measurement can be performed in a range of 0.5 to 10.0. When a forced expiration maneuver is measured, one breath is blown with PEFR selected, as shown in Fig. 9, a measured result 43a and a measuring mark 46 as lung function evaluation information showing which zone of green, yellow, and red the measured result 43a belongs to are displayed on the liquid crystal panel 4b. With decrease of the measured data of PEFR, the measuring mark 46 sequentially moves to be displayed on position of the color marks 40 printed on each zone, green zone, yellow zone, and red zone. Thus, by finding a zone displayed of the measuring mark 46, for example, a dosage depending on the patient's asthma condition is facilitated and coping suitably with the patient comes possible. In the drawing, "P" shows that the measured result is PEFR. In the drawing, "2" expresses that the expiration to be put into next is the second time one. When a select swicth 6 is depressed and FEV1 selected as shown, in Fig. 10, the FEV1 result 43b is displayed on the liquid crystal panel 4b. In the drawing, "S" designates that the measured result is FEV1. A reference value of the patient can previously be set depending on the patient by operation of the register switch 7 and the display switch 8.

With the register switch 7 depressed after several forced expirations are put at a time (in this embodiment, maximum three times) into the breath cylinder 2, the maximum value among the measured data is registered in the memory 23 described later. Even in depressing the register switch 7 after measuring at one time, the measured data is registered in the memory 23 described later.

The display switch 8 is provided for reading the past data 15c, and with the display switch 8 depressed as shown, in Fig. 11, morning data 43c on the corresponding day is displayed on the liquid crystal panel 4b. As shown in Fig.12, if the morning data 43c is not stored in the memory 23 described later, a mark 44, which shows that no data is present, is displayed on the liquid crystal panel 4b.

For the first shift switch 9, with the switch 9 depressed for the display in Fig. 11, the past data are scroll-displayed at every unit of morning, daytime, and evening as shown in Fig. 13.

For the second shift switch 10, with the switch 9 depressed for the display in Fig. 11, the past data are scroll-displayed at every date-unit basis ranging four weeks as shown in Figs. 14 and 15. The numerals 1 to 7 displayed on lower portion of the display, when they are lighted, designate the number of days passed since the data entry, that is, how many days ago the displayed past data 43c were enterd. As in the drawing, if "1" is lighted, this indicates the displayed past data 43c were entered one day ago. Numeral 47 appearing in center of the display designates the number of weeks passed since the data entry, that is, how many weeks ago the past data 43c were entered. As in the drawing, if "1" is lighted, this indicates the displayed past data 43c were entered one week ago.

Next, an operation of the breath flowmeter apparatus 1 of the present embodiment will be described. Assuming that the main body 3 is attached with the breath cylinder 2.

When the patient has a mouthpiece 201 in his mouth to expire his own breath into the breath cylinder 2, the expiratory flow detecting circuit 25 detects a current corresponding to the patient's expiratory flow rate. A detected value detected by the detecting circuit 25 is converted into digital data by the A/D converter 24 and thereafter sent to the CPU 20. The CPU 20 analyses PEFR and FEV1 from the detected value by the detecting circuit 25. Thus obtained measured data together with the measuring time information are stored and saved in the memory 23, and displayed on the display 4. For example, if PEFR is selected by operation of the select switch 6, then as shown in Fig. 9, a numeral information such as [326] (L/min) as PEFR is visually displayed. As described above, if the measurement of the expiration of the patient is performed, for example, three times per day (in the morning, the daytime, and evening), then every time the data are produced, the CPU 20 stores measured data of the patient's expiration and the measuring time information into the memory 23. The patient, after depressing the display switch 8, can scroll-display the past measured data and measuring time information on the display 4 as shown in Figs. 13 to 15 by operations of the first and second shift switches 9 and 10.

With the breath flowmeter apparatus 1 of the present embodiment, the following advantageous effects are obtained.
(1) The battery driving is employed, this enables portable type and facilitates measurement at home.
(2) The measured value of the expiration are displayed showing the zone the measured value of the expiration data belongs to, the zones consisting the green, yellow, and red zones each determined based on the reference value corresponding to the patient, thus the patient does not fail to recognize the displayed data and is capable of readily and objectively grasping the condition of the disease at the time of measuring. Therefore, a medical treatment at an early stage can be performed depending on the condition of the disease, thereby the treatment is made before the asthma attack occurs , and the patient is hardly required to enter the hospital with fast aid.
(3) Since recording the measured data in the diary etc. is unnecessary, and even children can easily measure the expiration data in their dally life including trip or going-out, a burden on the patient can be reduced.
(4) Since the operational results of PEFR and FEV1, which are entered several times per day for several weeks, can be stored together with the measuring time information for a given while the correspondence of the measuring time and the measured data is made clear, and the mis-recording or the data forging by the patient is prevented to ensure correct data.
(5) Since the maximum value among the operational results of a plurality of maneuvers is selected as PEFR or FEV1, stable measurements can be realized.
(6) Since FEV1 can be measured in addition to PEFR to enable evaluation as to whether or not the correct measurement has been conducted, an appropriate lung function evaluation can be obtained.
(7) Since the date and the time zone period when the measurement is conducted are displayedas the measuring time information, the measuring occasion is made clearer.
(8) Since the measured value and the lung function evaluation information are displayed as an operational result, a rapid diagnosis is realized.
(9) Since the measured data stored in the memory 23 is read on the host computer provided in the hospital to be analyzed, a burden on the medical doctor is reduced.
(10) Since the breath cylinder 2 can be removed from the main body 3, the breath cylinder 2 can be washed.

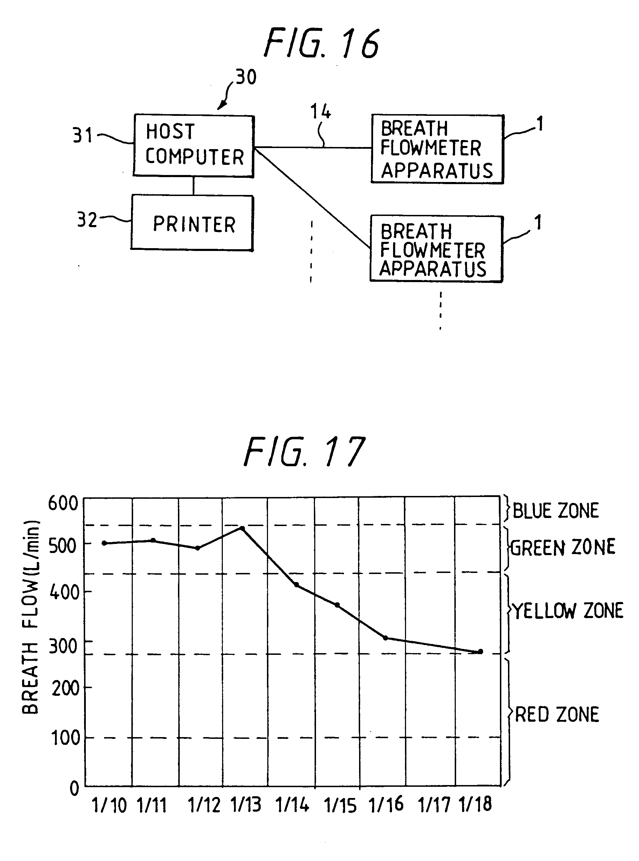

Fig. 16 is a schematic block diagram showing one embodiment of an asthma management system of the present invention.

The asthma management system of the embodiment has an asthma data management apparatus 30 controlling the entire system, the optional number of breath flowmeter apparatuses 1 are connected to the asthma data management apparatus 30 through an on-line 14 as a transmission means.

The asthma data management apparatus 30 comprises, as shown in Fig. 16, a host computer 31 as analysis means and a printer 32 as output means provided with a color printing function. The printer 32 outputs an analysis result by the host computer 31 as an analysis graph. A LCD (liquid crystal display) as output means may be used instead of the printer 32.

The host computer 31 performs analysis in accordance with a reference value at every patient basis. In detail, of green, yellow, and red zones are set corresponding to this reference value of the patient, and a trend graph of the measured PEFR, which is drawn on the zones, is output from the printer 22 as a analysis graph. Such output example is shown in Fig. 17. In the drawing, the vertical axis expresses the flow rate (L/min), the horizontal axis expresses date. Further the drawing shows a state where green zone, yellow zone, red zone and so on are displayed in respective colors in this order from the top.

Next, operations of the system of the present embodiment is described.

As formerly described, when the forced expiratory maneuver for the patient is performed using the breath flowmeter apparatus 1, for example, three times per day (morning, daytime, evening), then the measured data and measuring time information of expiration of the patient are stored into the memory 23 by the CPU 20.

Subsequently, after the patient measures the expiration for a predetermined period of time (for example, one week), the CPU 20 feeds the expiration data and the measuring time information stored in the memory 23 to the host computer 31 of the asthma data management apparatus 30 provided in the hospital through the communication interface 21 and the on-line 14. The host computer 31 reads the expiration data from the memory 23, analyzes the expiration data on the basis of the reference value on every patient basis , and outputs the analysis graph as shown in Fig. 16 from the printer 32. Such analysis graphical representation is provided for the diagnosis by the medical doctor.

The system may be configurated such that the asthma data management apparatus 30 and the breath flowmeter apparatus 1 are not always connected each other by the on-line 14, but both apparatuses 30 and 1 can be connected each other by a connecting cable. In this case, the patient carries the breath flowmeter apparatus 1 recorded with the expiration data to the hospital, and the analysis graph can be output, as formerly described, from the printer 32 by connecting through the connecting cable the asthma data management apparatus 30 placed in the hospital with the breath flowmeter apparatus 1 .

According to the asthma management system of the present embodiment, the advantageous effects are as follows.
(1) Since the analysis for the measured data can be performed by the asthma data management apparatus 30, a burden imposed on the medical doctor can be reduced, and the health maintenance for the bronchial asthmatic patient can securely be performed.
(2) The centralized control can be performed on the expiration data of a number of patients in the hospital.
(3) Although PEFR depends to effort of the patient, since FEV1 can also be measured, it is possible to evaluate whether or not the correct forced expiration has been done.

It is understood that in accordance with the present invention, various variation other than the embodiments described above can be made without departing from the subject matter of the invention. For example, in the above embodiments, PEFR and FEV1 are shown and described as a measuring item. However, the present invention is not limited to such embodiments described. When transmitting the expiration data from the breath flowmeter apparatus 1 to the asthma data management apparatus 30, recording cards such as IC cards and memory cards may be employed as a transmitting medium. In this case, a card reader for reading the expiration data from the recording card may preferably be provided on the asthma data management apparatus 30 side.

## Claims

1. A breath flowmeter apparatus comprising:
measuring means for measuring expiration data on the basis of an expiration into a breath breath cylinder at each measuring opearation;
displaying means for displaying the expiration data;
storage means for storing the expiration data together with measuring time information containing a date and a time period when the measurement is performed and;
a battery for supplying a power source to each part of the apparatus.

2. The breath flowmeter apparatus according to claim 1, wherein there is provided selecting means for selecting, where the measurements are performed by the measuring means a plurality of times during a same time period on a same date, an expiration datum indicative of maximum value out of thus obtained plurality of breath data as a datum of the time period so as to store thus selected datum.

3. The breath flowmeter apparatus according to claim 1 or claim 2, wherein the expiration data contain at least PEFR.

4. The breath flowmeter apparatus according to calim 1, claim 2 or claim 3, wherein the breath cylinder is configurated such that the itroducing cylinder is releasably mounted to the main body.

5. An asthma management system having a breath flowmeter apparatus and a asthma data management apparatus, characterized in that the breath flowmeter apparatus comprises a memory for storing expiration data measured at every measuring operation together with measuring time information containing a date and a time period when the measurement is performed, and transmitting means for transmitting the expiration data toghether with the time measuring information to the asthma data management apparatus, and that the asthma data management apparatus comprises anaylsis means for analyzing the expiration data transmitted by the transmitting means, and output means for outputting a analysis result by the analysis means.
